# EUROPEAN PATENT APPLICATION

(11) **EP 3 278 835 A1**
(43) Date of publication of application: **07.02.2018**
(21) Application number: 17184706.4
(22) Date of filing: 03.08.2017
(51) Int. Cl.: A61N 1/37, A61N 1/39, A61B 5/0402

(54) **METHOD FOR MONITORING THE STIMULATION FREQUENCY OF AN ICMD DEVICE**

(30) Priority: 04.08.2016 IT 201600082621
(71) Applicant: UNIVERSITA' DEGLI STUDI DI CAGLIARI, 09124 Cagliari (IT)
(72) Inventor: GATTO, GIANLUCA, 09127 Cagliari (IT); SERPI, ALESSANDRO, 09127 Cagliari (IT); SPANO, IVAN LUIGI, 09042 MONSERRATO (CA) (IT); LAI, ANDREA, 08048 TORTOLÌ (OG) (IT); NISSARDI, VINCENZO, 09127 Cagliari (IT); MARONGIU, IGNAZIO, 09127 Cagliari (IT)
(74) Representative: Giraldi, Elisa

(57) **Abstract**

Method for monitoring the stimulation frequency of an ICMD device as a cardiac signal changes, comprising the steps of a) determining (10) generation parameters for a reference cardiac signal; generating (12) said reference cardiac signal based on said parameters by progressively increasing its amplitude, at the end of successive acquisition intervals (H), until a maximum amplitude (Amax) is reached; comparing (18) the amplitude of a current cardiac signal with the maximum amplitude value (Amax) of the reference cardiac signal and, if the two values coincide, terminating the method; otherwise, increasing the amplitude of the reference cardiac signal by the predetermined value (ΔA) and repeating steps a)- c).

## Description

The present invention relates to a method for monitoring the stimulation frequency of an ICMD device as a cardiac signal changes.

In the last century, the research in the biomedical sector has had among the main objectives that one of render more manageable the life of the people and increase their duration.

In particular, we are currently living in an age in which many technical fields are becoming increasingly specialized in an attempt to find out solutions for preserving the quality of human life, thus passing from discovering new cells capable of neutralizing cancer to making specific studies for preventing genetic diseases, until the designing of special devices for supporting or even replacing human organs.

Such devices are also known as electromedical equipment. Among the available electromedical equipment, particular interest is aroused by those devices capable of monitoring the cardiac muscle, such as implantable cardiac medical devices (ICMD), among which the pacemaker (PMK) or the implantable cardioverter defibrillator (ICD). These devices have the task of assisting the heart, extinguishing the dangerous arrhythmias and preventing the cardiac arrest, which are, on a worldwide basis, one of the most frequent causes of death.

Considering the diffusion of heart diseases and the genetic correlation among them, demonstrated by scientific studies, the number of ICMD-dependent people is growing.

Given also the important task that these devices perform, it is essential that their operation ensures a full safety and precision in the delivery of therapies, in order to avoid malfunctions due to incorrect interpretation of the cardiac activity of the implanted patient, such as inappropriate interventions or inhibition of the device.

Such malfunctions may occur during the normal use of these devices, with the risk of damaging the human organism or even, in the worst-case scenario, leading it to death.

There is currently no (hardware or software) device capable of adequately verifying the behaviour of the pacing algorithm of an implantable cardiac device as cardiac intensity changes. The pacing algorithm is a functionality of any implantable cardiac medical device, and provides control over the monitored cardiac chamber through suitable stimulations, thanks to the use of specific electrocatheters. This algorithm is fundamental for the electric stimulation and contraction of the cardiac muscle, and is therefore indispensable both in pacemakers and in implantable defibrillators.

A known method generally concerning the field of monitoring the operation of ICMD devices is based on the detection of sensitivity thresholds, and is described in the CEI EN 45502-2-1 standard "*Part 2: Particular requiremehts for active implantable medical devices intended to treat bradyarrhythmia (cardiac pacemakers)*"*.*

The characterization described in said document is aimed at detecting the cardiac amplitude value at which the ICMD device switches from a stimulation state to the inhibition state.

However, such method only concerns the measurement of the sensitivity threshold; therefore, it does not provide, just like any other existing procedure or method, a characterization of the ICMD based on the identification of the inhibition process by monitoring the stimulation frequency thereof.

It is therefore one object of the present invention to propose an innovative method for monitoring the stimulation frequency of an ICMD device as a cardiac signal changes, which method allows overcoming the drawbacks of the prior art.

This and other objects are achieved through a method for monitoring the stimulation frequency of an ICMD device as a cardiac signal changes, the features of which are set out in claim 1.

Particular embodiments of the invention are set out in dependent claims, the contents of which should be interpreted as being an integral part of the present description.

Further features and advantages of the invention will be illustrated in the following detailed description, which is provided merely by way of non-limiting example with reference to the annexed drawings, wherein:

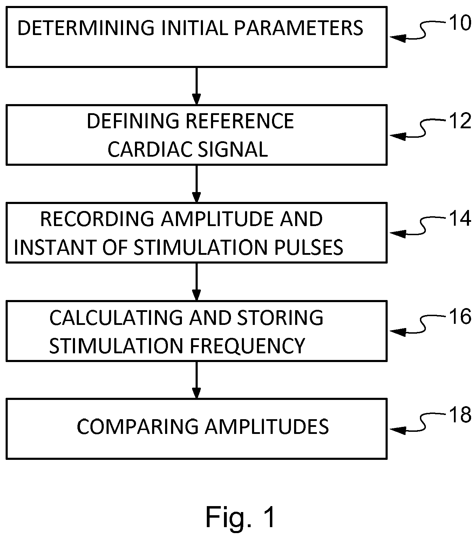

- Figure 1 is a block diagram of the steps of the method according to the present invention.

In brief, the method according to the present invention allows to execute the automatic monitoring of the stimulation frequency used by an ICMD device for responding to the detection of a cardiac signal, said ICMD device being intended to be subsequently implanted in a patient for effecting such stimulations upon the cardiac muscle of the patient.

The task of an implantable cardiac device is to assist the heart by means of appropriate stimulation pulses:, if for example it does not detect any cardiac activity (A=0), the ICMD generates and sends to the heart 60 stimulations per minute (60bpm), where A is the amplitude of the cardiac signal. The number of stimulations per minute is reduced as A increases, down to zero (completely inhibited ICMD) when the amplitude of the cardiac signal is sufficiently high (higher than or equal to a sensitivity threshold).

The method according to the present invention can be useful to manufacturers of implantable devices for verifying the proper operation thereof, even at cardiac frequency values where dangerous cardiac arrhythmias arise.

The method according to the present invention provides support for certifying the safe, robust and accurate operation of an ICMD device to be put on the market, i.e. suitable for implantation.

*In primis,* the present invention refers to the technical recommendations set out in the standards that describe the characterization procedures for implantable medical devices.

In the standards context, the most important standards are: CEI EN 45502-2-1 "*Part 2: Particular requirements for active implantable medical devices intended to treat bradyarrhythmia (cardiac pacemakers)"* and the standard CEI EN 45502-2-2 "*Part 2-2: Particular requirements for active implantable medical devices intended to treat tachyarrhythmia (includes implantable defibrillators)".*

In these standards there are described all the prescriptions that must be applied to implantable cardiac devices, and the tests, specified in CEI EN 45502-2-1, are considered as type tests.

Such tests must be conducted on samples of a specific ICMD device for the purpose of certifying the compliance thereof.

The description of the characteristics of the implantable pulse generator is of fundamental importance: they must be determined by using the different methods described in CEI EN 45502-2-1, or through any other method the accuracy of which has been demonstrated to be equal to or better than the accuracy specified in the standard.

The present invention takes into account the procedure called *"Sensitivity measurement (detection threshold) (epos and eneg)",* specified in the CEI EN 45502-2-1 standard, through which it is possible to measure the positive -epos-and negative -eneg- sensitivity thresholds that define the inhibition conditions of a device.

In this regard, said standard provides for measuring the sensitivity threshold only at one cardiac frequency value, obtainable by increasing the basic period of the implantable device by at least 50 ms.

The method according to the present invention allows evaluating and analyzing the functionalities of an ICMD device.

This method allows characterizing the operation of the ICMD device by verifying the number of stimulations that the pulse generator can send to the cardiac muscle.

The method according to the present invention is bound to the generation of a cardiac signal having the characteristics specified in the CEI EN 45502-2-1 standard.

The method according to the invention, being based on a system for acquisition and generation of electric signals, allows monitoring the number of stimulations effected by an ICMD device within a predetermined observation time interval H.

Figure 1 shows a block diagram of the steps of the method according to the present invention. The method comprises two main phases.

The first phase comprises a step 10 of determining initial parameters, based on which the method has be continued. More specifically, this step defines a time interval H for the acquisition of stimulations effected by an ICMD device in response to a reference cardiac signal generated for stimulating the cardiac activity for the purpose of executing the method of the present invention, a frequency FH of a predetermined reference cardiac signal, and minimum (Amin) and maximum (Amax) amplitude values that said reference cardiac signal will take during the process.

The second phase is the core of the method.

In a step 12 said reference cardiac signal is defined and, throughout the acquisition time interval H, said cardiac signal is generated, which has an amplitude A that changes progressively within the interval comprised between Amin and Amax.

In particular, an initial reference cardiac signal having an amplitude Amin is generated, and said signal is then increased, at the end of a first acquisition time interval H, by a predetermined amplitude increment value ΔA; subsequently, a reference cardiac signal having an amplitude Amin + ΔA is generated, until the end of a second acquisition time interval H, at which time the amplitude of the reference cardiac signal is increased again by the amplitude increment value ΔA, and so on until the maximum amplitude value Amax is reached.

The frequency of said reference cardiac signal is always equal to FH, and said signal is characterized by the properties described in the CEI EN 45502-2-1 standard.

During the execution of the method according to the present invention, the reference cardiac signal is only modified in its amplitude, without changing the time characteristics t and T described and specified in the CEI EN 45502-2-1 standard.

In a subsequent step 14, for each amplitude value of the reference cardiac signal, the instantaneous amplitude and the time instant of the stimulation pulses generated by the ICMD device as a function of the perceived cardiac signal are both recorded.

The amplitude and the time instant at which each stimulation pulse is effected by the ICMD device are thus recorded and, on the basis of this information, a stimulation frequency FE (as described below) is calculated and stored for each amplitude value, starting from Amin up to Amax.

Subsequently, in step 16, the stimulation frequency FE of the ICMD device is calculated in a *per se* known manner and stored, starting from a total number of stimulations measured within a predetermined time interval, e.g. one minute.

At the end of every observation interval H, in step 18 the amplitude of the current cardiac signal (generated in a *per se* known manner) is compared in a *per se* known manner, e.g. by means of a comparator, with the maximum value Amax specified for the reference cardiac signal: if the two values coincide, the method will end; otherwise, the amplitude of the cardiac signal will be increased by a quantity ΔA and a new cycle of the method will begin.

At the end of the method according to the present invention, a data set will be available about the stimulation frequency FE for each amplitude value of the reference cardiac signal.

This information will highlight, after having been processed in a *per se* known manner, which lies outside the method of the present invention, the relationship between the stimulation frequency of the ICMD device and the amplitude of the cardiac signal of the patient.

This relationship is particularly interesting for studying the properties of the ICMD inhibition process and for detecting and signalling any anomalies in the programming of the device.

Of course, without prejudice to the principle of the invention, the forms of embodiment and the implementation details may be extensively varied from those described and illustrated herein by way of non-limiting example, without however departing from the protection scope of the present invention as set out in the appended claims.

## Claims

1. Method for monitoring the stimulation frequency of an ICMD device as a cardiac signal changes, comprising the steps of:
a) determining (10) generation parameters for a reference cardiac signal;
b) generating (12) said reference cardiac signal based on said parameters by progressively increasing its amplitude, at the end of successive acquisition intervals (H), until a maximum amplitude (Amax) is reached;
c) comparing (18) the amplitude of a current cardiac signal with the maximum amplitude value (Amax) of the reference cardiac signal and, if the two values coincide, terminating the method; otherwise, increasing the amplitude of the reference cardiac signal by the predetermined value (ΔA) and repeating steps a)- c).

2. Method according to claim 1, wherein the step of determining (10) generation parameters for a reference cardiac signal comprises determining:
a time interval for the acquisition of stimulations (H) effected by the ICMD device in response to a reference cardiac signal generated for stimulating the cardiac activity;
a reference frequency (FH) of said reference cardiac signal;
values of minimum amplitude (Amin) and maximum amplitude (Amax) of said reference cardiac signal.

3. Method according to claim 2, wherein the step of generating (12) said reference cardiac signal comprises the steps of defining said reference cardiac signal having an initialization amplitude equal to the minimum amplitude (Amin) and a frequency equal to the reference frequency (FH), and generating said reference cardiac signal by progressively increasing the amplitude, at the end of successive acquisition intervals (H), by a predetermined value (ΔA) until the maximum amplitude (Amax) is reached.

4. Method according to any of the preceding claims, further comprising the step of recording (14), for each amplitude value of the reference cardiac signal, both the instantaneous amplitude and the time instant of stimulation pulses generated by the ICMD device as a function of the reference cardiac signal, and calculating (16) a stimulation frequency (FE) of the ICMD device starting from a total number of stimulations measured within a predetermined time interval.
